# EUROPEAN PATENT APPLICATION

(11) **EP 2 336 771 A1**
(43) Date of publication of application: **22.06.2011**
(21) Application number: 09015552.4
(22) Date of filing: 16.12.2009
(51) Int. Cl.: G01N 33/53, G01N 33/574

(54) **HAS3v2 as tumor marker**

(71) Applicant: Universität Duisburg-Essen, 45141 Essen (DE)
(72) Inventor: Fischer, Jens, Prof. Dr., 40629 Düsseldorf (DE); Dai, Guang, Dr., Bao Tou City Inner Mongolia (CN); Mengede, Christian, Dr., 45768 Marl (DE)
(74) Representative: Roth, Carla

(57) **Abstract**

The present invention pertains to the field of tumor medicine. In particular, a splice variant of hyaluronan synthase 3, HAS3v2, has been identified as tumor marker useful for diagnosis and prognosis of specific cancers, especially esophageal adenocarcinomas. The present invention furthermore provides means for detecting HAS3v2 such as specific antibodies.

## Description

### FIELD OF THE INVENTION

The present invention pertains to the field of tumor medicine. In particular, a splice variant of hyaluronan synthase 3, HAS3v2, has been identified as tumor marker useful for diagnosis and prognosis of specific cancers, especially esophageal adenocarcinomas. The present invention furthermore provides means for detecting HAS3v2 such as specific antibodies.

### BACKGROUND OF THE INVENTION

Hyaluronan (HA) is a linear polysaccharide belonging to the family of glucosominoglycans (GAGs). It consists of alternating N-acetylglucosamine and D-glucuronic acid units linked by β(1-4) and β(1-3) bonds. In average, hyaluronan consists of approximately 25,000 disaccharide units and can range in size from 5 to 20,000 kDa *in vivo.* HA is synthesized by enzymes named hyaluronan synthases and degraded by hyaluronidases. The turnover of HA in the human body occurs rapidly. Around one third of hyaluronan in the human body is turned over each day.

In human, three different hyaluronan synthases (HAS) genes have been found, named HAS1, HAS2 and HAS3. Recently, it was revealed that human HAS3 has two splicing variants, designated HAS3v1 and HAS3v2 [Sayo, T. et al- (2002) The Journal of Investigative Dermatology 118, 43-48; Monslow, J. et al. (2003) The International Journal of Biochemistry & Cell Biology 35, 1272-1283]. Northern blot analysis showed two bands of 4.8 kb and 2 kb, resulting from alternative splicing processes. The HAS enzymes are localized in the plasma membrane comprising seven membrane domains. Site directed mutagenesis experiments showed that the active center of HAS likely resides in the large intracellular loop between the second and the third transmembrane domain. The hyaluronan synthesized by the HAS enzymes is transported to the extracellular space, forming a part of the extracellular matrix (ECM).

In comparison to HAS3v1 (553 aa), the splicing variant HAS3v2 is shorter (281 aa) and lacks the five C-terminal membrane domains. The first 246 amino acid from HAS3v1 and HAS3v2 are identical, including the enzymatically active intracellular domain. On gene level, HAS3v2 comprises a different 5'-UTR and a different last exon coding for the C-terminal amino acids. However, in contrast to HAS3vl, the biological and pathological role of the splice variant HAS3v2 is yet unknown.

HA has diverse functions despite of its unbranched simple composition. HA binds a huge amount of water in the extracellular space and thus hydrates tissues and skin dermis. Hyaluronan regulates water balance, osmotic pressure and acts as an ion exchange resin. Furthermore, HA forms a thin layer in the luminal surface of blood vessel, named apical glycocalyc that excludes certain macromolecules, leukocytes and platelets from direct interaction with the luminal surface of endothelial cells. As a lubricant and a shock absorber, hyaluronan acts as a structural molecule in the vitreous humor of the eye, in joint fluid, and in Wharton's jelly. Furthermore, it is now known that HA plays an important role in modulating cell behavior. Hyaluronan regulates cell motility, cell-cell and cell-matrix adhesion, cell proliferation and differentiation. It participates in fundamental processes such as embryological development and morphogenesis, wound healing, repair and regeneration and inflammation. HA regulates the intracellular signaling pathways by binding to the proteins known as hyaladherins, including LYVE1 (Lymphatic Vessel Endothelial Receptor 1), HARE (Hyaluronan Receptor for Endocytosis), toll-like receptors TLR and TLR4, CD44 (Cluster of Differentiation 44) and RHAMM (Receptor for Hyaluronan Mediated Motility).

Hyaluronan is shown to be elevated in both the cancer epithelium and peritumoral stroma during cancer progression depending on the cancer type. Either is associated with an unfavorable outcome of the disease. Both HAS1 and HAS2 expression are elevated in highly malignant cells transformed with v-src. Furthermore, overexpression of HAS2 and HAS3 genes resulted in HA over-production and increased tumorigenicity of fibrosarcoma, melanoma, and mesothelioma cells. The importance of HAS enzymes for tumor cell phenotypes is also supported by the finding that suppression of HAS2 or HAS3 diminished the tumorigenic potential in a variety of tumors. Elevated HAS1 expression and intronic gene splicing correlate with poor prognosis in human colon cancer, ovarian cancer, and multiple myelomas. Although the above evidences strongly supports the pro-tumorigenic role of HAS, the tumor promoting ability of HAS upregulation can not be generalized because HAS2 overexpression can e.g. also inhibit the tumorigenesis of glioma cells. Hyaluronan positive cancer cells were detected in about half of the breast and prostate tumor cases and over 80 % of ovarian, colorectal and gastric cancers showed hyaluronan positive cancer. In most cases, hyaluronan is detected around the plasma membrane. However, other studies showed that accumulation of hyaluronan in tumor could serve as a favorable prognostic factor. In lung squamous cell carcinoma, de-differentiated cancer cells showed less HA staining compared to higher differentiated ones with a more promising prognosis. Therefore, the role of hyaluronan and its association with progression and metastasis cannot be generalized and needs to be studied in different cancer entities.

Esophageal cancer is the 3^{rd} most common gastrointestinal malignancy and is the 6^{th} most frequent cancer worldwide. The highest incidence of the disease occurs in the Esophageal Cancer Belt which stretches from eastern Turkey to Iraq, Iran, China, India, and certain regions of South Africa. More than 90 % of the esophageal cancers are either esophageal squamous cell carcinomas (ESCC) or esophageal adenocarcinomas (EAC). Although patient presentation of ESCC and EAC are similar, the epidemiology, etiology, prognosis and treatment strategy are quite different. ESCC and EAC are considered to represent two different diseases occurring in the same organ. In view of this, suitable markers for identifying the type of esophageal cancer are helpful for their diagnosis and treatment.

One object of the present invention was the provision of a tumor marker suitable for diagnosing specific cancer, in particular EAC.

### SUMMARY OF THE INVENTION

The present inventors have now found that HAS3v2 is highly expressed in specific cancer types such as esophageal adenocarcinomas compared to the corresponding normal tissue such as normal mucosa tissue. Therefore, HAS3v2 can be used as marker for diagnosing, monitoring, staging and/or prognosing cancer and/or the metastatic behavior of cancer in a patient.

In this respect, the present invention in a first aspect provides a method for diagnosing, monitoring, staging and/or prognosing cancer and/or the metastatic behavior of cancer in a patient, comprising the step of analyzing the expression of HAS3v2 in a biological sample isolated from said patient.

In a second aspect, the present invention provides a method for discriminating between esophageal adenocarcinoma and esophageal squamous cell carcinoma, comprising the step of analyzing the expression of HAS3v2 in the carcinoma.

In a third aspect, the present invention provides the use of HAS3v2 as marker for diagnosing, monitoring, staging and/or prognosing cancer and/or the metastatic behavior of cancer in a patient.

In a fourth aspect, the present invention provides a binding agent which is capable of specifically binding to HAS3v2 protein, in particular an antibody or an antigen-binding fragment thereof. The binding agent can be used for detecting HAS3v2 protein in a sample, for example in the methods according to the first and second aspect of the invention.

In a fifth aspect, the present invention provides a kit for performing the method according to the first and/or the second aspect of the invention, comprising at least one agent suitable for analyzing the expression of HAS3v2 and instructions for use.

Other objects, features, advantages and aspects of the present invention will become apparent to those skilled in the art from the following description and appended claims. It should be understood, however, that the following description, appended claims, and specific examples, which indicate preferred embodiments of the application, are given by way of illustration only. Various changes and modifications within the spirit and scope of the disclosed invention will become readily apparent to those skilled in the art from reading the following.

### DETAILED DESCRIPTION OF THE INVENTION

### Definitions:

As used herein, the following expressions are generally intended to preferably have the meanings as set forth below, except to the extent that the context in which they are used indicates otherwise.

The expression "comprise", as used herein, besides its literal meaning also includes and specifically refers to the expressions "consist essentially of" and "consist of'. Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of' and "consist of'.

A "binding agent" may be any compound or complex or compounds which is capable of binding a target substance such as HAS3v2 protein. Preferably, the binding agent is capable of specifically binding the target substance. Suitable binding agents may be obtained by screening a binding agent library in order to identify/obtain binding agents that bind to the target substance. In preferred embodiments, the candidate binding agents may further be screened against the binding to a non-target substance such as HAS3v1. Examples for respective binding agent libraries are for example phage or phagemid libraries, which display the binding agents. Methods for obtaining e.g. antibodies *in vitro* are also described by Hudson and Souriau (Hudson, P.J. and Souriau, C. (2003) "Engineered antibodies" Nat. Med. 9, 129-134).

The binding agents may have any structure, as long as they are able to specifically recognize and bind the target substance. Binding agents may be selected from the group consisting of antibodies, antigen-binding fragments or variants thereof having, binding agents having a protein scaffold providing a binding function such as for example anticalins. An overview of binding agents which have a similar binding function as antibodies is given in Hey, et al. (Hey et al. (2005) "Artificial, non-antibody binding proteins for pharmaceutical and industrial application", Trends in Biotechnology 23(10), 514-522). An antibody variant is a derivative of an antibody or antibody fragment having the same binding function but e.g. an altered amino acid sequence.

The term "antibody" particularly refers to a protein comprising at least two heavy chains and two light chains connected by disulfide bonds. The term "antibody" includes naturally occurring antibodies as well as all recombinant forms of antibodies, e.g., antibodies expressed in prokaryotes, non-glycosylated antibodies, humanized antibodies, and chimeric antibodies. Each heavy chain is comprised of a heavy chain variable region (V_{H}) and a heavy chain constant region (C_{H}), Each light chain is comprised of a light chain variable region (V_{L}) and a light chain constant region (C_{L}). The heavy chain-constant region comprises three or - in the case of antibodies of the IgM- or IgE-type - four heavy chain-constant domains (C_{H}1, C_{H}2, C_{H}3 and C_{H}4) wherein the first constant domain C_{H}1 is adjacent to the variable region and may be connected to the second constant domain C_{H}2 by a hinge region. The light chain-constant region consists only of one constant domain. The variable regions can be further subdivided into regions of hypervariability, termed complementarity determining regions (CDRs), interspersed with regions that are more conserved, termed framework regions (FR), wherein each variable region comprises three CDRs and four FRs. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

An "antigen-binding fragment" of an antibody in particular is a protein or glycoprotein which is derived from said antibody and is capable of binding to the same antigen, in particular to the same epitope as the antibody. Thus, an antigen-binding fragment of an antibody herein generally refers to a functional fragment. Preferably, the antigen-binding fragment comprises at least 20 amino acids from said whole antibody, more preferably at least 100 amino acids, preferably including the binding region of the antibody. In particularly preferred embodiments, the antigen-binding fragment of an antibody comprises a heavy chain variable region. It has been shown that the antigen-binding function of an antibody can be performed by fragments of a full-length antibody. Examples of fragments of an antibody include (i) Fab fragments, monovalent fragments consisting of the variable region and the first constant domain of each the heavy and the light chain; (ii) F(ab)₂ fragments, bivalent fragments comprising two Fab fragments linked together, for example by a disulfide bridge at the hinge region; (iii) Fd fragments consisting of the variable region and the first constant domain C_{H}1 of the heavy chain; (iv) Fv fragments consisting of the heavy chain and light chain variable region of a single arm of an antibody; (v) scFv fragments, Fv fragments consisting of a single polypeptide chain; (vi) (Fv)₂ fragments consisting of two Fv fragments covalently linked together; (vii) a heavy chain variable domain; and (viii) multibodies consisting of a heavy chain variable region and a light chain variable region covalently linked together in such a manner that association of the heavy chain and light chain variable regions can only occur intermolecular but not intramolecular. These antibody fragments are obtained using conventional techniques known to those with skill in the art.

"Specific binding" preferably means that an agent such as an antibody binds stronger to a target such as an epitope for which it is specific compared to the binding to another target. An agent binds stronger to a first target compared to a second target if it binds to the first target with a dissociation constant (K_{d}) which is lower than the dissociation constant for the second target. Preferably the dissociation constant for the target to which the agent binds specifically is more than 2-fold, preferably more than 5-fold, more preferably more than 10-fold, even more preferably more than 20-fold, 50-fold, 100-fold, 200-fold, 500-fold or 1000-fold lower than the dissociation constant for the target to which the agent does not bind specifically. If an agent which specifically binds to a target compound (such as HAS3v2 protein or nucleic acid coding for HAS3v2) is used in a particular method for binding to said target compound, the conditions for binding preferably are chosen so that binding of said agent to other, in particular similar compounds (such as other HAS proteins like HAS3v1 protein or nucleic acid coding for other HAS like HAS3v1) is not detectable in the method or does not interfere with the detection and/or determination of the binding to the target compound. With respect to the binding to HAS3v2 protein, "specific binding" in particular means that the agent binds stronger to HAS3v2 protein than to another HAS protein, in particular HAS3v1 protein. With respect to the binding to a nucleic acid coding for HAS3v2, "specific binding" in particular means that the agent binds stronger to a nucleic acid coding for HAS3v2 than to a nucleic acid coding for another HAS, in particular HAS3v1.

As used herein, the term "protein" refers to a molecular chain of amino acids or a complex of more than one amino acid chain. A protein can contain any of the naturally occurring amino acids as well as artificial amino acids and can be of biologic or synthetic origin. A protein may be modified, naturally (post-translational modifications) or synthetically, by e.g. glycosylation, amidation, carboxylation and/or phosphorylation. A protein comprises at least two amino acids, but does not have to be of any specific length; this term does not include any size restrictions, In the present application, the terms "protein", "polypeptide" and "peptide" are used interchangeably. Preferably, a protein comprises at least 10 amino acids, preferably at least 50 amino acids, at least 100 amino acids and most preferred at least 100 amino acids.

The term "nucleic acid" indudes single-stranded and double-stranded nucleic acids, including ribonucleic acids as well as deoxyribonucleic acids. It may comprise naturally occurring as well as synthetic nucleotides and can be naturally or synthetically modified, for example by methylation, 5'- and/or 3'-capping.

The expression "determining the level of expression" of HAS3v2 preferably relates to the determination of the presence or absence and/or the absolute and/or relative quantification of HAS3v2 mRNA and/or HAS3v2 protein. Any suitable method for detecting and/or quantifying nucleic acids or proteins can be used. Examples of methods for detecting and/or quantifying nucleic acids are PCR, in particular real time PCR, and hybridization assays such as Northern blot, dot blot or RNAse protection assays. Methods for detecting and/or quantifying proteins include, for example, immunologic assays such ELISA, RIA, Western blot and immunohistochemistry.

According to the invention, the term "increased" preferably refers to an increase by at least 10%, in particular at least 20%, at least 50% or at least 100%. The amount of a substance is also increased in a test sample such as a biological sample compared to a reference sample if it is detectable in the test sample but absent or not detectable in the reference sample.

According to the invention, a "reference" such as a reference sample or reference organism may be used to correlate and compare the results obtained in the methods of the invention from a test sample or test organism, i.e. a patient. Typically the reference organism is a healthy organism, in particular an organism which does not suffer from cancer and/or metastasis of cancer.

A "detectably labeled" compound according to the invention preferably refers to a compound coupled to a label which provides a detectable signal such as a luminescent, fluorescent or phosphorescent label, a radioactive label or a FRET (fluorescence resonance energy transfer) label, or an affinity label which is capable of binding to a further compound which provides a detectable signal.

The term "patient" means according to the invention a human being, a nonhuman primate or another animal, in particular a mammal such as a cow, horse, pig, sheep, goat, dog, cat or a rodent such as a mouse or rat. In a particularly preferred embodiment, the patient is a human being.

The term "cancer" according to the invention in particular comprises leukemias, seminomas, melanomas, teratomas, lymphomas, neuroblastomas, gliomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, head and neck cancer, gastrointestinal cancer, lymph node cancer, esophagus cancer, colorectal cancer, pancreas cancer, ear, nose and throat (ENT) cancer, breast cancer, prostate cancer, cancer of the uterus, ovarian cancer and lung cancer, and the metastases thereof.

By "tumor" is meant a group of cells or tissue that is formed by misregulated cellular proliferation. Tumors may show partial or complete lack of structural organization and functional coordination with the normal tissue, and usually form a distinct mass of tissue, which may be either benign or malignant.

By "metastasis" is meant the spread of cancer cells from its original site to another part of the body. The formation of metastasis is a very complex process and normally involves detachment of cancer cells from a primary tumor, entering the body circulation and settling down to grow within normal tissues elsewhere in the body. When tumor cells metastasize, the new tumor is called a secondary or metastatic tumor, and its cells normally resemble those in the original tumor. This means, for example, that, if breast cancer metastasizes to the lungs, the secondary tumor is made up of abnormal breast cells, not of abnormal lung cells. The tumor in the lung is then called metastatic breast cancer, not lung cancer.

According to the invention, "staging" of a cancer preferably refers to the classification of the progression and extent of a cancer. A preferred cancer staging system is the TNM classification of malignant tumors, wherein T describes the size of the tumor and whether it has invaded nearby tissue, N describes regional lymph nodes that are involved, and M describes distant metastasis. Each of these parameters is given a particular value depending on the situation in the patient, wherein generally a higher number indicates a more severe situation (T(0-4), N(0-3), M(0/1)). Additionally, for a more detailed classification further parameters can be determined and/or prefixes can be used. Furthermore, the TNM classification may be summarized in a cancer staging system according to the UICC, referring to cancer of from stage 0 to stage lV.

According to the invention, a biological sample may be a tissue sample, including bodily fluids, and/or a cellular sample and may be obtained in the conventional manner such as by tissue biopsy, including punch biopsy, and by taking blood, bronchial aspirate, sputum, urine, feces or other body fluids. According to the invention, the term "biological sample" also includes fractions of biological samples.

The present invention is based on the finding of the inventors that HAS3v2 expression is increased in specific cancer cells compared to normal, non-cancerous cells. This could be demonstrated, in particular, for esophageal adenocarcinomas (EAC) wherein the HAS3v2 expression is increased more than 10-fold in comparison to normal mucosa cells. Therefore, HAS3v2 expression levels can be used as marker for specific cancers such as EAC. Furthermore, it has been found that HAS3v2 expression is not increased in esophageal squamous cell carcinoma (ESCC) compared to normal mucosa. Thus, the expression of HAS3v2 can be used as marker to discriminate between EAC and ESCC.

Additionally, the present inventors have demonstrated that HAS3v2 expression is higher in tumors having a higher cancer stage. Therefore, the expression level of HAS3v2 can also be used to support the staging of tumors and/or to determine the metastatic potential of a tumor.

The above findings are even more surprising as it has been found that HAS3v2 protein when expressed in cells is localized intracellularly, in particular at the membrane of the endoplasmic reticulum and/or the Golgi apparatus. This, since to date only the role of extracellular hyaluronan (HA) in various cancers was discussed. However, the enzymatic activity of HAS3v2 results in the production of intracellular HA. In summary, the present inventors have found that HAS3v2 can be used as marker for diagnosing, monitoring, staging and/or prognosing cancer and/or the metastatic behavior of cancer in a patient.

In view of the above described findings, the present invention provides a method for diagnosing, monitoring, staging and/or prognosing cancer and/or the metastatic behavior of cancer in a patient, comprising the step of analyzing the expression of HAS3v2 in a biological sample isolated from said patient. The same method may be used for diagnosing, monitoring, staging and/or prognosing a tumor and/or the metastatic behavior of a tumor in a patient. If in the following it is referred to cancer, the described features and embodiments likewise can be applied to the method for diagnosing, monitoring, staging and/or prognosing a tumor and/or the metastatic behavior of a tumor.

The analysis of the expression of HAS3v2 preferably comprises quantitatively and/or qualitatively determining the level of expression of HAS3v2, in particular the quantitative determination thereof. The level of expression determined in the biological sample can then be used as indication for the presence and/or amount of cancerous cells in the sample, for the stage of the cancer, for the metastatic potential and/or behavior of the cancer, and/or for the prognosis of the cancer. Preferably, this involves the comparison of the determined level of expression of HAS3v2 with a reference value. The reference value may be, for example, a standard value of the level of expression of HAS3v2 in normal tissue, in particular normal mucosa tissue, or a standard value of the level of expression of HAS3v2 in cancer cells, in particular of a cancer of the same tissue as the tissue in the biological sample. Alternatively, the reference value may be the level of expression of HAS3v2 determined in another biological sample, preferably from the, same patient. This further biological sample preferably does not contain cancer tissue and preferably comprises non-cancer cells originating from that tissue from which also the first sample is taken. This further biological sample may also be obtained from the same tissue of the same patient, but at an earlier date, e.g. for monitoring a cancer disease and/or its treatment. Furthermore, the reference value may be the level of expression of HAS3v2 determined in a biological sample taken from a subject without cancer or from a subject without metastasis of cancer, preferably having a similar cancer, or from a subject with a similar cancer, preferably with a known stage of cancer.

In preferred embodiments, an increase of the level of expression of HAS3v2 in the biological sample compared to the reference sample is indicative for the presence of cancer, for the presence of metastasis of cancer, or for a more advanced stage of cancer. Depending on what information is to be obtained by the method according to the invention, the reference sample is chosen. For example, a subject without cancer is chosen as reference if the presence of cancer is to be determined; a subject without metastasis of cancer, but preferably with cancer, is chosen as reference if the presence of metastasis of cancer is to be determined; and a subject with cancer, preferably with a known stage of cancer, is chosen as reference if the stage of cancer is to be determined.

The level of expression of HAS3v2 can be determined, for example, either on the mRNA level or on the protein level. Thus, determination of the level of expression of HAS3v2 preferably comprises detecting or determining the amount of a nucleic acid coding for HAS3v2, and/or detecting or determining the amount of HAS3v2 protein.

Preferably, the detection or determination of the amount of HAS3v2 nucleic acid comprises specifically amplifying said nucleic acid and determining the amount of the amplification product, and/or specifically binding said nucleic acid to a hybridization probe and determining the amount of a complex between the nucleic acid and the hybridization probe. In preferred embodiments, the level of expression of HAS3v2 is determined by determining the amount of HAS3v2 mRNA in the biological sample, for example using PCR, in particular real time PCR. In particular, the determination may involve lysis of the sample, isolation of mRNA and amplification of the HAS3v2 mRNA or a part thereof under controlled conditions. Standard protocols and commercially available kits and apparatuses can be used. Alternatively, the level of expression of HAS3v2 can be determined by hybridizing a sequence specific nucleic acid probe to the HAS3v2 mRNA. This can be performed directly in the obtained biological sample or after isolation of the mRNA. Preferably, the hybridization probe is detectably labeled and/or a nucleic acid. Suitable detectable labels are described above and known to the person skilled in the art.

The detection or determination of the amount of HAS3v2 protein preferably comprises contacting the biological sample with an agent which binds specifically to said protein and detecting the formation of or determining the amount of a complex between the agent and the protein. The agent may be any compound which specifically binds to the HAS3v2 protein, however, antibodies specific for HAS3v2 are preferred. Thus, the agent may be any antibody or antigen-binding fraction thereof or any further protein binding agent as described herein which is capable of specifically binding HAS3v2. Preferably, the affinity of the antibody to the other HAS proteins, in particular HAS3v1, is much lower than its affinity to HAS3v2, i.e. the dissociation constant of these interactions is much higher than that of the interaction with HAS3v2. For detecting the formation of or determining the amount of a complex between the agent and the HAS3v2 protein, preferably a detectable label is used. The detectable label may be coupled to the agent or a further compound bearing the detectable label is used which is capable of binding to the agent. Suitable detectable labels are described above and known to the person skilled in the art. Furthermore, any known method for detecting or determining the amount of a specific protein can be used in the method according to present invention. Examples of suitable methods are described above. In particular embodiments, the detection or determination of the amount of HAS3v2 protein comprises the detection or determination of HAS3v2 protein fragments such as degradation products. In particular, peptide fragments of HAS3v2 proteins which are presented by MHC molecules on the surface of the cells may be detected or their amount may be determined. Preferably, these peptide fragments are specific for HAS3v2 and the processing or degradation of other HAS proteins such as HAS3v1 in the cell does not result in the presentation of similar or identical peptide fragments on MHC molecules.

In certain embodiments, analyzing the expression of HAS3v2 may comprise the analysis of markers or signals which are indicative for the rate of production of HAS3v2 mRNA. Examples of such markers or signals are cellular signals which influence the splicing of the HAS3 prepro-mRNA so that the production of HAS3v2 mRNA is favored over the production of HAS3v1 mRNA, or mutations or modifications of the HAS3 gene which promote the splicing of the transcription product into the HAS3v2 mRNA.

In particular preferred embodiments, the cancer which is to be diagnosed, monitored, staged and/or prognosed is an esophageal adenocarcinoma. However, the cancer may be any cancer which has an increased level of expression of HAS3v2 when compared to the level of expression of HAS3v2 in the corresponding normal tissue. Such cancers may be, for example, esophageal cancer, leukemias, seminomas, melanomas, teratomas, lymphomas, neuroblastomas, gliomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, head and neck cancer, gastrointestinal cancer, lymph node cancer, colorectal cancer, pancreas cancer, ear, nose and throat (ENT) cancer, breast cancer, prostate cancer, cancer of the uterus, ovarian cancer and lung cancer, and the metastases thereof. In particular, the cancer may be an adenocarcinoma such as an adenocarcinoma of the colon, lung, cervix, prostate, urachus, vagina, breast, esophagus, pancreas or stomach.

Preferably, the patient is a human being. Furthermore, the HAS3v2 preferably is human HAS3v2. In particular, HAS3v2 mRNA preferably has the nucleic acid sequence according to SEQ ID NO: 1, and/or HAS3v2 protein preferably has the amino acid sequence according to SEQ ID NO: 2.

Furthermore, the present invention provides a method for discriminating between esophageal adenocarcinoma and esophageal squamous cell carcinoma, comprising the step of analyzing the level of expression of HAS3v2 in the carcinoma. Again, the analysis of the expression of HAS3v2 preferably comprises quantitatively and/or qualitatively determining the level of expression of HAS3v2, in particular the quantitative determination thereof. In particular, an increased level of expression of HAS3v2 in the carcinoma compared to the level of expression of HAS3v2 in normal mucosa cells is indicative for an esophageal adenocarcinoma. Likewise, a similar level of expression of HAS3v2 in the carcinoma and in normal mucosa cells is indicative for an esophageal squamous cell carcinoma. Preferably, the level of expression of HAS3v2 is determined in a biological sample of a carcinoma found in a patient. Furthermore, the normal mucosa cells used for comparison are preferably obtained from the same patient. However, the level of expression of HAS3v2 may alternatively be compared to another reference such as a standard reference obtained from analysis of one or more different biological samples, for example samples containing mucosa cells of a subject without cancer, or samples containing esophageal adenocarcinoma cells or samples containing esophageal squamous cell carcinoma cells. The specific embodiments described above for the method according to the first aspect of the invention also correspondingly apply to this method according to the second aspect of the invention, where appropriate.

In the above described methods, the binding of HAS3v2 protein with a specific binding agent such as a HAS3v2-specific antibody is a tool for determining the level of expression of HAS3v2. However, prior to the present invention researchers and technicians have faced serious problem when trying to provide antibodies against HAS proteins. Regarding HAS3v2, one commercial antibody was available prior to the present invention. However, this antibody did not show specific binding in experimental assays, demonstrating the difficulties in generating specific antibodies suitable for *in* vivo as well as *in vitro* applications. The present inventors now have generated an antibody which is specific for HAS3v2 and is functional in *in vivo* and in *vitro* assays.

Therefore, in a further aspect, the present invention provides a binding agent which is capable of specifically binding to HAS3v2 protein. In particular, the binding agent is capable of specifically binding to an epitope formed by the amino acids at position 247 to 261 (SEQ ID NO: 3) and/or 268 to 281 (SEQ ID NO: 4) of human HAS3v2 or a part thereof. The binding agent preferably is a protein, in particular an antibody, an antigen-binding fragment thereof or an anticalin. The antibody may be polyclonal or monoclonal. Preferably, the binding agent according to the invention is capable of binding to HAS3v2 in *vivo,* in particular to HAS3v2 in its natural three-dimensional structure and/or at its natural location in the cell. Furthermore, the binding agent preferably is capable of binding to HAS3v2 *in vitro,* in particular to HAS3v2 in its denatured structure. The binding agent preferably is specific for human HAS3v2, in particular HAS3v2 having the amino acid sequence of SEQ ID NO: 2. The antibody, antigen-binding fragment thereof or anticalin may be from any organism, but preferably is or is derived from a human, mouse or rat antibody or anticalin. The binding agent according to the invention may be used in the methods according to the invention, in particular for detecting or determining the amount of HAS3v2 protein in a biological sample.

The present invention furthermore provides a kit for performing the methods according to the invention, comprising at least one agent suitable for analyzing the expression of HAS3v2 and instructions for use. The agent suitable for analyzing the expression of HAS3v2 preferably is an agent capable of specifically binding to HAS3v2 mRNA or cDNA or a binding agent capable of specifically binding to HAS3v2 protein. The agent capable of specifically binding to HAS3v2 mRNA or cDNA may be any compound, in particular nucleic acid, which specifically binds to HAS3v2 mRNA or cDNA, resepctively. Particular examples thereof are nucleic acid molecules which are at least partially complementary to the HAS3v2 mRNA or cDNA. Preferably, these nucleic acid molecules are capable of binding to the HAS3v2 mRNA or cDNA under stringent conditions, and more preferably do not bind to other HAS mRNA or cDNA, in particular HAS3v1 mRNA or cDNA, under stringent conditions. Stringent conditions are described, for example, in "Molecular Cloning: A Laboratory Manual", J. Sambrook et al., 2nd Edition, Cold Spring Harbor Laboratory Press, Cold Spring Harbor, New York, 1989 or "Current Protocols in Molecular Biology", F.M. Ausubel et al., John Wiley & Sons, Inc., New York. The binding agent capable of specifically binding to HAS3v2 protein preferably is a binding agent as described herein, in particular an antibody, an antigen-binding fragment thereof or an anticalin specific for HAS3v2. The instructions for use preferably contains a protocol for performing the method for diagnosing, monitoring, staging and/or prognosing cancer and/or the metastatic behavior of cancer according to the invention and/or the method for discriminating between esophageal adenocarcinoma and esophageal squamous cell carcinoma according to the invention.

### FIGURES

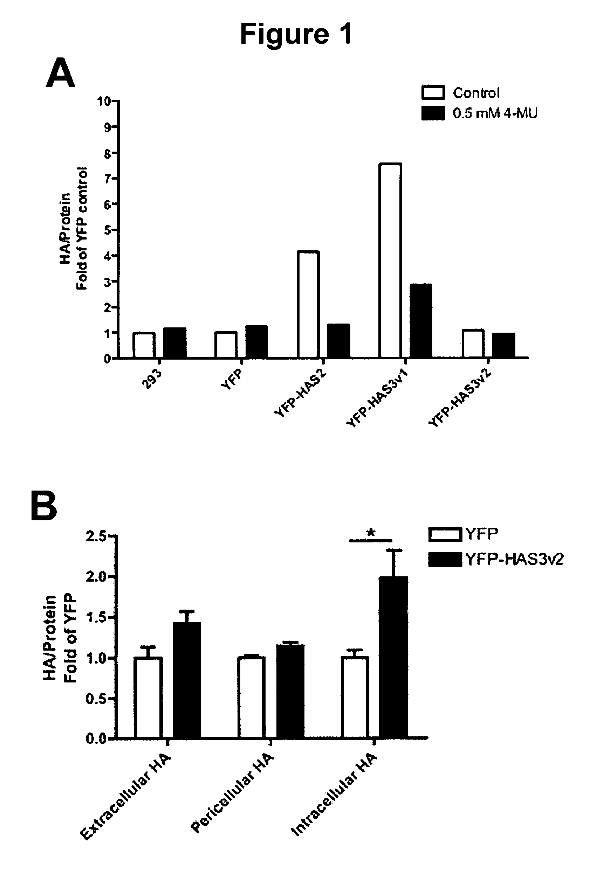

**Figure 1****:** Quantification of extra-, peri- and intracellular HA after YFP-HAS transfections. (A) After transfection of HEK 293 cells, HA concentration was determined from cell culture supernatant and normalized to cellular protein. In addition, 4-MU was used to inhibit HA synthesis. Data are normalized to YFP control. White bar without 4-MU; black bar: with 0.5 mM 4-MU: representative result out of triplicate experiment.: HAS3v2 increases intracellular HA. HAS2 and HAS3v1 expression result in an increased HA concentration in the culture medium, while HAS3v2 expression does not increase the extracellular HA concentration. (B) Extra-, peri- and intracellular HA from stablely transfected HEK cells was quantified by HA ELISA. HAS3v2 expression leads to a markedly increased intracellular HA concentration.

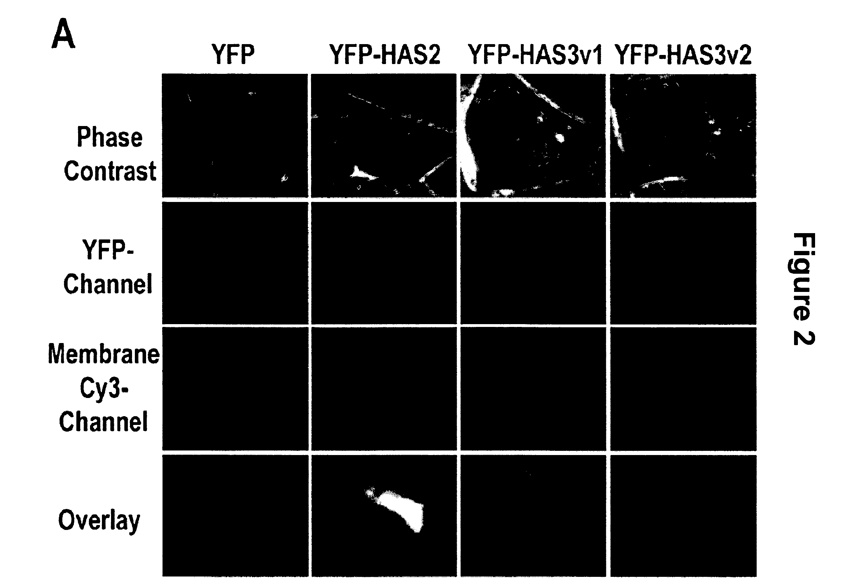

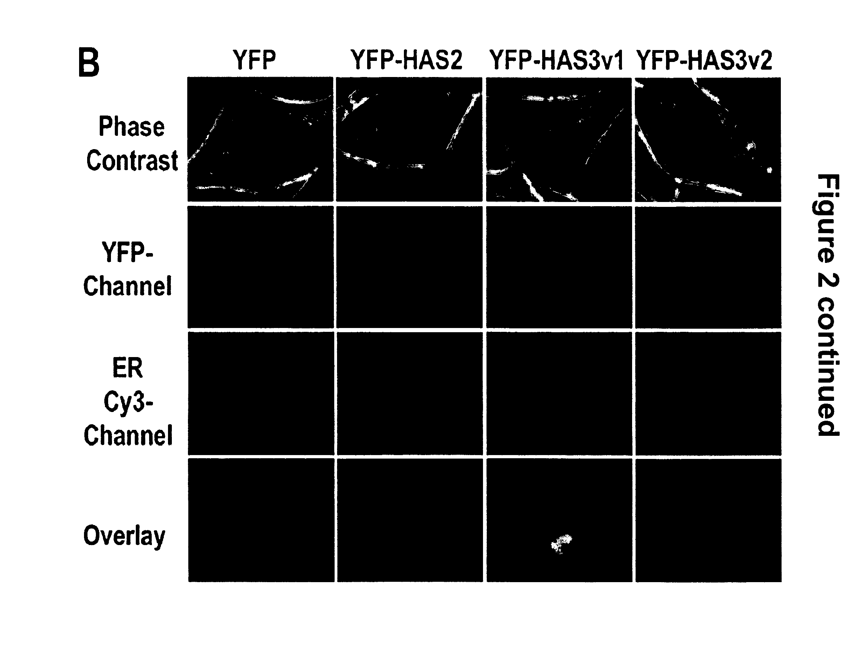

**Figure 2****:** Co-localization analysis of YFP fusion proteins of different HAS proteins using plasma membrane and ER markers. (A) Cy3 conjugated plasma membrane marker; (B) Cy3 conjugated ER marker. Magnification: 400 x. Co-localization of the plasma membrane marker and HAS2 or HAS3v1 can be seen, while HAS3v2 colocalizes with the ER marker but not with the plasma membrane marker.

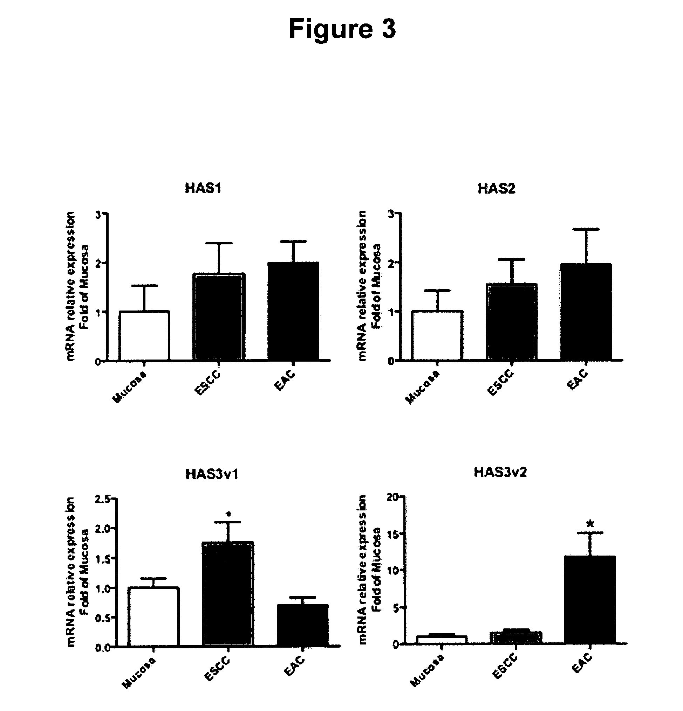

**Figure 3****:** Expression level of HAS isoforms in human ESCC and EAC compared to mucosa. mRNA expression was determined by RT-qPCR from tumor material removed during surgery. As control, healthy mucosal tissue from the surrounding area of the excised tumors was used. One way ANOWA method was used for statistic analysis. n = 13 for mucosa control, n = 12 for ESCC and n = 40 for EAC, respectively. An about 12-fold higher HAS3v2 mRNA expression can be seen in EAC compared to ESCC and mucosa tissue.

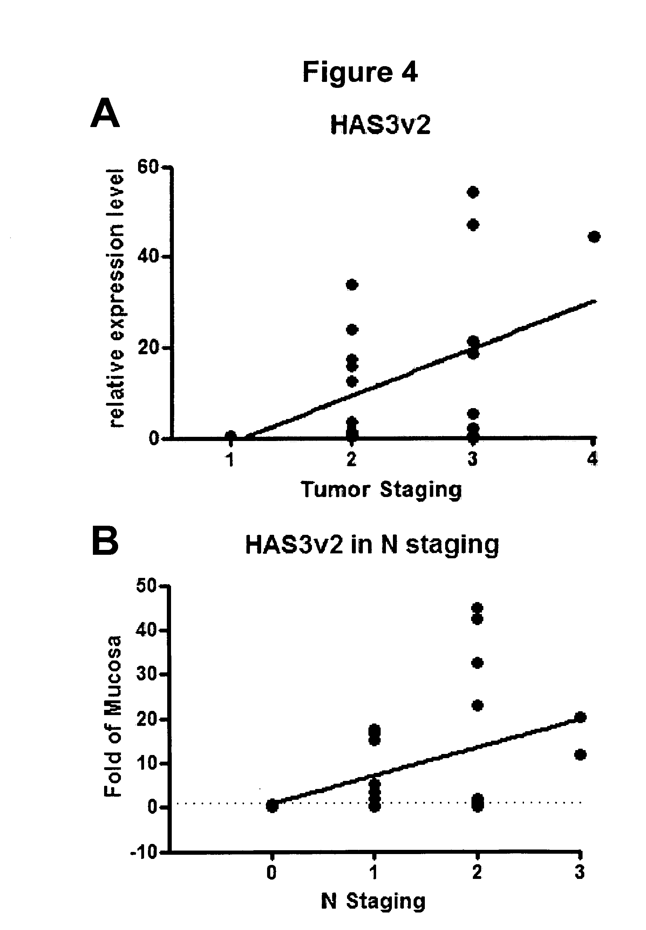

**Figure 4****:** HAS3v2 expression level is positively correlated with EAC TNM tumor staging mRNA expression was determined by RT -qPCR from tumor material removed during surgery. As control, healthy mucosal tissue from the surrounding area of the excised tumors was used. Correlation coefficient method was used for statistic analysis. (A) Correlation with tumor staging; n = 5 for T1, n = 16 for T2, n = 17 for T3, and n = 2 for T4. (B) Correlation with lymph note staging. An increasing HAS3v2 expression is correlated with increasing TNM tumor stage.

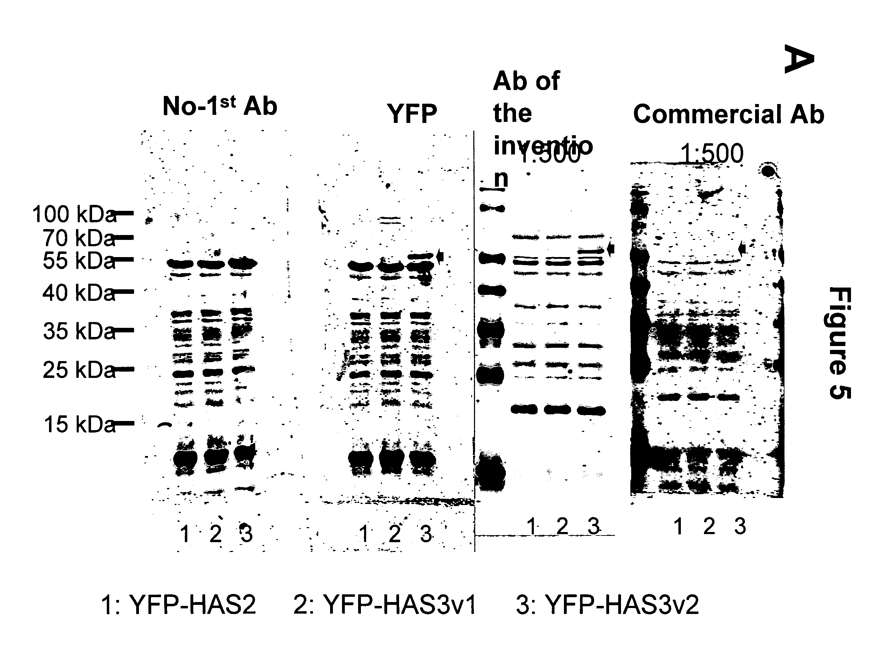

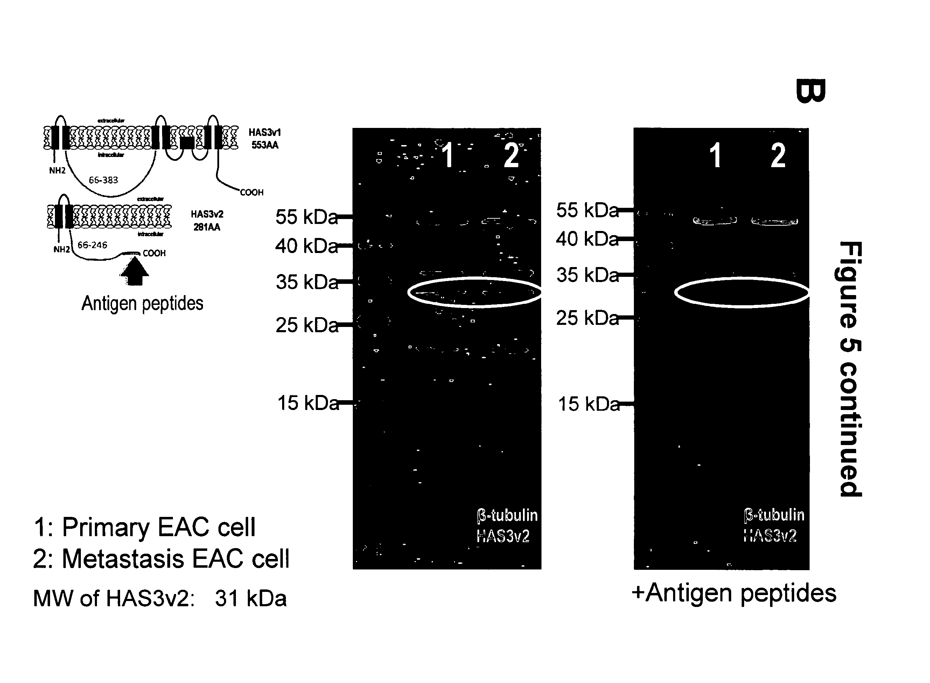

**Figure 5****:** The antibody of the invention specifically binds HAS3v2. (A) Western blot analysis of cells transfected with YFP-HAS2 (90 kDa, lane 1), YFP-HAS3v1 (90 kDa, lane 2) or YFP-HAS3v2 (58 kDa, lane 3). In the first blot, no primary antibody was used to show the unspecific signals of the secondary antibody used. In the second blot, an anti-YFP antibody was used to show fusion protein expression. The third blot clearly shows the 58 kDa YFP-HAS3v2 protein detected with the anti-HAS3v2 antibody of the invention. In the third blot, where the commercially available anti-HAS3v2 antibody was used, HAS3v2 is not visible. Thus, only the anti-HAS3v2 antibody of the invention is capable of binding to HAS3v2 while the commercial antibody is not functional. (B) Western blots of lysates of primary (line 1) and metastatic (line 2) EAC cells with the anti-HAS3v2 antibody of the invention in the absence and presence of the antigen peptides used for generating the antibody were performed. HAS3v2 could be detected in primary and metastatic EAC cells. The HAS3v2 signal in the metastatic EAC cells is significantly stronger than that in the primary EAC cells, indicating a higher HAS3v2 expression in metastatic EAC. This specific interaction could be inhibited by addition of the antigen peptides.

### EXAMPLES

### Example 1: Subcellular localization of HAS3v2

To address the subcellular localization of HAS3v2, YFP fusion proteins were constructed. The CMV promoter was used to drive the expression of YFP-HAS fusion proteins. RT-PCR was performed to obtain the full-length coding sequences of human HAS2, HAS3v1 and HAS3v2 from total RNA of human smooth muscle cells. Afterwards, these sequences were cloned to fuse onto the 3' end of the YFP coding sequence. The YFP-HAS chimera were over-expressed by transfecting the target cells with the constructed YFP fusion protein expression vectors. The mock YFP expression vector served as control. HEK 293 cells were selected as the host, in which YFP, YFP-HAS2, YFP-HAS3v1 and YFP-HAS3v2 stablely overexpressed. The stable cell clones were selected by puromycin beginning 24 hours after transfection. Colonies were picked after 7 days of selection. The culture medium from these stable cell lines were collected for measuring HA secretion within 24 hours after medium change. As is shown in figure 1 (A), overexpression of YFP-HAS2 and YFP-HAS3v1 induced extracellular HA up to 7 fold within 24 hours compared to YFP mock control. The overproduction of HA could be attenuated by a specific inhibitor of hyaluronan synthases, namely 4-methylumbelliferone (4-MU). This indicates that the YFP-HAS fusion proteins are active. Overexpressing YFP-HAS3v2 in HEK cell did not lead to increased secretion of HA in the cell culture medium. However, HAS3v2 intracellularly synthesizes HA, resulting in an increase in intracellular, but not peri- or extracellular HA concentration, as shown in figure 1 (B).

The sub-cellular expression patterns of the YFP-HAS fusion proteins were examined by fluorescent microscopy. A plasma membrane marker was introduced to evaluate whether HAS3v2 is localized in the plasma membrane. As shown in figure 2 (A), in case of YFPHAS3v1 and YFP-HAS2 the YFP signal surrounding the cell overlapped with the Cy3 signal from the plasma membrane marker. However, there was no overlap between the YFP signal from YFP-HAS3v2 and the plasma membrane marker. This illustrates that, unlike HAS2 and HAS3v1, HAS3v2 is not present in the plasma membrane. The YFP mock control shows ubiquitous signal inside the cell. A marker for the endoplasmic reticulum (Cy3-ER) was utilized for the co-localization experiment. In figure 2 (B), the YFP mock control showed overlap with the ER-Golgi marker, which was expected due to the ubiquitous localization inside the cell. Also, partial overlap of the ER marker and the accumulated intracellular YFP signal from YFP-HAS2 and YFP-HAS3v1 was observed. The phase contrast pictures revealed that the condensed intracellular YFP signal of both YFP-HAS2 and YFP-HAS3v1 can be tracked to the golgi apparatus. This might be explained by the fact that both HAS2 and HAS3v1 are processed in the golgi and further transported to the plasma membrane. Importantly, the subcellular interconnected network-like expression pattern of HAS3v2 showed a complete overlap with the ER marker, which suggested that HAS3v2 is indeed localized in the ER.

### Example 2: Upregulation of HAS3v2 In esophageal adenocarcinoma (EAC)

Biopsies were collected from patients who suffered from either esophageal squamous cell carcinoma (ESCC) or esophageal adenocarcinoma (EAC). After the surgical intervention, samples were stored immediately in liquid nitrogen. Total RNA was isolated and the relative expression levels of hyaluronan synthase isoforms were quantified by realtime RT qPCR. All the data were then normalized to the average of the mucosa group. As shown in figure 3, HAS3v1 is upregulated in ESCC compared to mucosa and other HAS isoforms remain constant. In contrast, only HAS3v2 was significantly upregulated in the ADC group compared to mucosa.

Next, it was examined whether the induction of HAS3v2 was correlated with tumor progression. Tumor stage and grading were classified by routine histopathologic assessment according to the UICC (Union Internationale Contre le Cancer) Classification for Malignant Tumors. Then, the relative expression levels of HAS3v2 from each tumor sample were plotted against the respective TNM tumor staging. As is shown in figure 4, HAS3v2 expression correlates with increasing tumor staging. Correlation coefficient method was used for statistic analysis.

### Example 3: Preparation and analysis of an anti-HAS3v2 antibody

A polyclonal anti-HAS3v2 antibody was prepared by standard procedures. Briefly, mice were immunized with the HAS3v2 specific peptides PPGKGMAVEDDQVQA (SEQ ID NO: 3), corresponding to amino acids 247 to 261 of human HAS3v2, and EAWSVHQRHVSREQ (SEQ ID NO: 4), corresponding to amino acids 268 to 281 of human HAS3v2. Then, the produced anti-HAS3v2 serum was obtained from the mice and used in Western blot analysis to assess the ability of the polyclonal antibody to bind HAS3v2. It could be shown that this antibody binds to HAS3v2. Furthermore, this binding is specific as it can be inhibited by addition of the antigen peptides used for raising the antibody (figure 5 (B)). However, a commercially available antibody against HAS3v2 was not able to bind to HAS3v2 (figure 5 (A)). Thus, the anti-HAS3v2 antibody of the invention is the first antibody which indeed is able to specifically bind to HAS3v2.

## Claims

1. A method for diagnosing, monitoring, staging and/or prognosing cancer and/or the metastatic behavior of cancer in a patient, comprising the step of analyzing the expression of HAS3v2 in a biological sample isolated from said patient.

2. The method of claim 1, wherein an increased level of expression of HAS3v2 compared to a subject without cancer is indicative for cancer, and/or an increased level of expression of HAS3v2 compared to a subject without metastasis of cancer is indicative for a metastatic behavior of cancer, and/or an increased level of expression of HAS3v2 compared to a subject with a similar cancer is indicative for a more advanced stage of cancer.

3. The method of claim 1 or 2, wherein the cancer is an esophageal adenocarcinoma.

4. A method for discriminating between esophageal adenocarcinoma and esophageal squamous cell carcinoma, comprising the step of analyzing the expression of HAS3v2 in the carcinoma.

5. The method according to claim 4, wherein an increased level of expression of HAS3v2 in the carcinoma compared to the expression of HAS3v2 in normal mucosa cells is indicative for an esophageal adenocarcinoma.

6. The method of any one of claims 1 to 5, wherein the step of analyzing the expression of HAS3v2 comprises quantitatively determining the level of expression of HAS3v2.

7. The method of any one of claims 1 to 6, wherein the step of analyzing the expression of HAS3v2 comprises detecting or determining the amount of a nucleic acid coding for HAS3v2, and/or detecting or determining the amount of HAS3v2 protein in the biological sample.

8. Use of HAS3v2 as marker for diagnosing, monitoring, staging and/or prognosing cancer and/or the metastatic behavior of cancer in a patient.

9. A binding agent which is capable of specifically binding to HAS3v2 protein.

10. The binding agent of claim 9, being an antibody, an antigen-binding fragment thereof or an anticalin which preferably is capable of specifically binding to an epitope formed by the amino acids at position 247 to 261 and/or 268 to 281 of human HAS3v2 or a part thereof.

11. A kit for performing the method according to any one of claims 1 to 7, comprising at least one agent suitable for analyzing the expression of HAS3v2 and instructions for use.

12. The kit of claim 11, wherein the agent suitable for analyzing the expression of HAS3v2 is an agent capable of specifically binding to HAS3v2 mRNA or cDNA or the binding agent according to claim 9 or 10.

13. The kit of claim 11 or 12, wherein the instructions for use contain instructions for performing the method according to any one of claims 1 to 7 using the kit.


SEQUENCE LISTING

<110>  Universität Duisburg-Essen

<120>  HAS3v2 as tumor marker

<130>  51 229 K

<160>  4

<170>  PatentIn version 3.3

<210>  1
<211>  1229
<212>  DNA
<213>  Homo sapiens

<400>  1
ggaggggcat ggagccgccg cgggcctgct gagctccgga gcgcggcagc cggcggcacg      60

atgccggtgc agctgacgac agccctgcgt gtggtgggca ccagcctgtt tgccctggca     120

gtgctgggtg gcatcctggc agcctatgtg acgggctacc agttcatcca cacggaaaag     180

cactacctgt ccttcggcct gtacggcgcc atcctgggcc tgcacctgct cattcagagc     240

ctttttgcct tcctggagca ccggcgcatg cgacgtgccg gccaggccct gaagctgccc     300

tccccgcggc ggggctcggt ggcactgtgc attgccgcat accaggagga ccctgactac     360

ttgcgcaagt gcctgcgctc ggcccagcgc atctccttcc ctgacctcaa ggtggtcatg     420

gtggtggatg gcaaccgcca ggaggacgcc tacatgctgg acatcttcca cgaggtgctg     480

ggcggcaccg agcaggccgg cttctttgtg tggcgcagca acttccatga ggcaggcgag     540

ggtgagacgg aggccagcct gcaggagggc atggaccgtg tgcgggatgt ggtgcgggcc     600

agcaccttct cgtgcatcat gcagaagtgg ggaggcaagc gcgaggtcat gtacacggcc     660

ttcaaggccc tcggcgattc ggtggactac atccaggtgt gcgactctga cactgtgctg     720

gatccagcct gcaccatcga gatgcttcga gtcctggagg aggatcccca agtaggggga     780

gtcgggggag atgtccagcc cccagggaaa ggtatggcag tagaggatga ccaggtccaa     840

gctgcccagg tcagagctac ggaagcatgg tccgttcacc aacgccacgt ttctagagag     900

cagtgagctg attctccaat ggtgagcagg ggactacatg tgaactggga cctgcaggcc     960

aatgtatccc tgaggaaaag tccacaagaa caattcaaga aactagtaag aaacaatggg    1020

cagaaagctg tgggacgaaa gcacagcagg cttctgggag gctggagatc ctttctctca    1080

agggcaggat tattcccacc tgccacagtt cacatgccac aaataacatc tcaccttcag    1140

tcaagaaaaa cgcaaaggaa aaaaaaaaaa aaaaaaaaaa aaaaaaaaaa aaaaaaaaaa    1200

aaaaaaaaaa aaaaaaaaaa aaaaaaaaa                                      1229

<210>  2
<211>  281
<212>  PRT
<213>  Homo sapiens

<400>  2

Met Pro Val Gln Leu Thr Thr Ala Leu Arg Val Val Gly Thr Ser Leu
1               5                   10                  15

Phe Ala Leu Ala Val Leu Gly Gly Ile Leu Ala Ala Tyr Val Thr Gly
            20                  25                  30

Tyr Gln Phe Ile His Thr Glu Lys His Tyr Leu Ser Phe Gly Leu Tyr
        35                  40                  45

Gly Ala Ile Leu Gly Leu His Leu Leu Ile Gln Ser Leu Phe Ala Phe
    50                  55                  60

Leu Glu His Arg Arg Met Arg Arg Ala Gly Gln Ala Leu Lys Leu Pro
65                  70                  75                  80

Ser Pro Arg Arg Gly Ser Val Ala Leu Cys Ile Ala Ala Tyr Gln Glu
                85                  90                  95

Asp Pro Asp Tyr Leu Arg Lys Cys Leu Arg Ser Ala Gln Arg Ile Ser
            100                 105                 110

Phe Pro Asp Leu Lys Val Val Met Val Val Asp Gly Asn Arg Gln Glu
        115                 120                 125

Asp Ala Tyr Met Leu Asp Ile Phe His Glu Val Leu Gly Gly Thr Glu
    130                 135                 140

Gln Ala Gly Phe Phe Val Trp Arg Ser Asn Phe His Glu Ala Gly Glu
145                 150                 155                 160

Gly Glu Thr Glu Ala Ser Leu Gln Glu Gly Met Asp Arg Val Arg Asp
                165                 170                 175

Val Val Arg Ala Ser Thr Phe Ser Cys Ile Met Gln Lys Trp Gly Gly
            180                 185                 190

Lys Arg Glu Val Met Tyr Thr Ala Phe Lys Ala Leu Gly Asp Ser Val
        195                 200                 205

Asp Tyr Ile Gln Val Cys Asp Ser Asp Thr Val Leu Asp Pro Ala Cys
    210                 215                 220

Thr Ile Glu Met Leu Arg Val Leu Glu Glu Asp Pro Gln Val Gly Gly
225                 230                 235                 240

Val Gly Gly Asp Val Gln Pro Pro Gly Lys Gly Met Ala Val Glu Asp
                245                 250                 255

Asp Gln Val Gln Ala Ala Gln Val Arg Ala Thr Glu Ala Trp Ser Val
            260                 265                 270

His Gln Arg His Val Ser Arg Glu Gln
        275                 280

<210>  3
<211>  15
<212>  PRT
<213>  Artificial

<220>
<223>  antigen peptide

<400>  3

Pro Pro Gly Lys Gly Met Ala Val Glu Asp Asp Gln Val Gln Ala
1               5                   10                  15

<210>  4
<211>  14
<212>  PRT
<213>  Artificial

<220>
<223>  antigen peptide

<400>  4

Glu Ala Trp Ser Val His Gln Arg His Val Ser Arg Glu Gln
1               5                   10